# EUROPÄISCHE PATENTSCHRIFT

(11) **EP 0 930 911 B1**
(45) Veröffentlichungstag und Bekanntmachung des Hinweises auf die Patenterteilung: **23.11.2005**
(21) Anmeldenummer: 98945182.8
(22) Anmeldetag: 07.08.1998
(51) Int. Cl.: A61M 25/01, A61B 5/028

(54) **KATHETERSYSTEM**
CATHETER SYSTEM
SYSTEME DE CATHETER

(30) Priorität: 07.08.1997 DE 19734220
(43) Veröffentlichungstag der Anmeldung: 28.07.1999
(73) Patentinhaber: Pulsion Medical Systems AG, 81829 München (DE)
(72) Erfinder: PFEIFFER, Ulrich, D-81667 München (DE)
(74) Vertreter: Kehl, Günther
(86) Internationale Anmeldenummer: PCT/EP1998/005033
(87) Internationale Veröffentlichungsnummer: WO 1999/007429

(56) Entgegenhaltungen:
- EP-A- 0 266 928
- EP-A- 0 719 519
- US-A- 5 279 573
- US-A- 5 443 457
- US-A- 5 514 092

## Beschreibung

### Gebiet der Erfindung

Die vorliegende Erfindung betrifft ein Kathetersystem mit einem Einführungsdraht und mit einem Katheter, bei dem im distalen Katheterspitzenbereich ein Einführungsdrahtlumen vorgesehen ist, das sich von der Katheterspitze bis zu einer seitlich im Katheterspitzenbereich vorgesehenen Öffnung erstreckt.

### Beschreibung des Standes der Technik

Die Plazierung von Kathetern über kleine Blutgefäße wird derzeit im wesentlichen mittels einer aus zwei verschiedenen Verfahren kombinierten Technik durchgeführt:

Zur Überwachung des arteriellen Blutdrucks und der arteriellen Blutgase insbesondere im perioperativen Bereich in der Anästhesie wird heutzutage als Standardverfahren die Arteria radialis herangezogen. In der Regel werden hierbei einlumige intravasale Katheter aus Teflon oder aus Polyurethan mit einer Länge zwischen 3 cm und 5 cm und mit einem Durchmesser von bis zu 18 Gauge in die Arteria radialis eingeführt.

Die Katheter werden hierbei üblicherweise über eine Führungskanüle, die das Lumen des Katheters ausfüllt und diesen überragt, eingeführt. Darüber wird meist mittels Flüssigkeitskopplung an einen externen Druckaufnehmer der arterielle Druck invasiv gemessen. Blutgase und andere physiologische Parameter werden aus Blutproben bestimmt, die über das Lumen des Katheters entnommen werden.

Als Nachteil der bisherigen Punktionstechnik der Arteria radialis ist in diesem Zusammenhang aufzuführen, daß es durch das Einführen eines oben beschriebenen Katheters in das Gefäßlumen vielfach zu einer reaktiven Konstriktion der Arteria radialis und damit zu einem Sistieren des Blutflusses am Katheter kommt, wodurch auch falsche Druckmessungen zustande kommen (Mohr R., Lavee J., Goor D.A. (1987): Inaccuracy of radial artery pressure measurement after cardiac operations. J Thorac Cardiovascular Surg, 94(2): 286-290). Dieser fälschlicherweise als korrekt arteriell interpretierte Druck entsteht, indem eine pulsierende stagnierende Blutsäule gemessen wird.

Wird über das Druck-/Blutentnahmelumen des Katheters Blut abgenommen, so stimmen biochemische oder physiologische Werte dieser Probe vielfach nicht mit der augenblicklichen Situation überein, da dieses Blut zumindest teilweise aus einer am Kreislauf nicht partizipierenden Blutsäule stammt. Analoge Falschmessungen ergeben sich, wenn ein relativ kurzer Radialiskatheter mit Sensoren für biochemische oder physiologische Parameter (beispielsweise Blutgase) versehen ist.

Druckkurven in der Arteria radialis weichen in der Regel sowohl in der Form als auch im systolischen und diastolischen Blutdruck stark von in zentralen Arterien, das heißt in der Aorta gemessenen Druckkurven ab (Gravlee G.P., Brauer S.D., O'Rourke M.F., Avolio A.P. (1989): A comparison of brachial, femoral, and aortic intra-arterial pressures before and after cardiopulmonary bypass. Anaesth Intensive Care, 17(3): 305-311). Gerade in kritischen Situationen ist jedoch eine Kenntnis der zentralen arteriellen Druckkurven erforderlich.

Peripher-arterielle Druckkurven aus der Arteria radialis sind auch nicht zur Berechnung des Schlagvolumens des Herzens nach der Pulskonturmethode (Wesseling K.H., de Wit B., Weber A.P., Ty Smith N. (1983): A Simple Device for the Continuous Measurement of Cardiac Output. Adv Cardiovasc Phys, vol 5 (Part II): 16-52) geeignet, da eine eindeutige Diagnose der Auswurfphase der linken Herzkammer nicht möglich ist. Hierzu werden zentral gemessene Druckkurven benötigt.

Das Herzzeitvolumen und der Kreislauffüllungszustand werden im perioperativen Bereich meist über einen - zusätzlich zum arteriellen Radialiskatheter - plazierten pulmonalarteriellen Thermodilutionskatheter (Synonym: Pulmonaliskatheter, Pulmonalarterienkatheter) bestimmt. Bei der pulmonalarteriellen Thermodilution wird zentralvenös eine Glukose- oder Kochsalzlösung injiziert, die eine von der Bluttemperatur abweichende Temperatur aufweist. Der Pulmonalarterienkatheter weist am distalen Ende - plaziert in der Arteria pulmonalis - einen Temperatursensor auf, mit dem die Thermodilutionskurve registriert wird. Aus dieser Thermodilutionskurve wird beispielsweise mittels des Stewart-Hamilton-Verfahrens das Herzzeitvolumen berechnet. Des weiteren wird durch Aufblasen eines am distalen Ende des Katheters plazierten Ballons ein pulmonalarterieller Verschlußdruck registriert, der eine Information über den Kreislauffüllungszustand geben soll.

Zwar können das Herzzeitvolumen und der Kreislauffüllungszustand auch mittels der transkardiopulmonalen, arteriell gemessenen Thermodilution (Pfeiffer U.J., Knoll R. (1993): Process for Determining a Patient's Circulatory Fill Status. United States Patent No. 5,526,817) oder auch mittels der Thermo-Dye-Dilution (Pfeiffer U.J., Backus G., Blümel G., Eckart J., Müller P., Winkler P., Zeravik J., Zimmermann G.J. (1990): A Fiberoptics-Based System for Integrated Monitoring of Cardiac Output, Intrathoracic Blood Volume. Extravascular Lung Water, 0₂ Saturation, and a-v Differences. Practical Applications of Fiberoptics in Critical Care Monitoring, Springer Verlag, 114-125) gemessen werden, doch gestatteten die bisherigen Meßkatheter lediglich die zuverlässige Messung in der Arteria femoralis bzw. in der Aorta abdominalis.

Die Erfahrung zeigt, daß die bisher entwickelten arteriellen Thermodilutions- und Thermo-Dye-Dilutionskatheter mit Blutdrucklumen mit einem kleinsten Durchmesser von 1,33 mm immer noch keine zuverlässige Thermo- oder Thermo-Dye-Dilutionsmessung bei Punktion der Radialarterie erlauben, da diese nur über einen Einführkatheter mit deutlich größerem Außendurchmesser plaziert werden können. Für eine Anwendung während der Anästhesie ist aber eine Radialismessung erforderlich, weil der Anästhesist während vieler Operationen keine Möglichkeit hat, einen Femoraliskatheter bezüglich seiner Lage zu korrigieren, oder weil infolge des durchgeführten operativen Eingriffs kein Blutfluß in der Arteria femoralis vorhanden ist oder dieser durch Einflußnahme des Chirurgen verfälscht ist.

Als Nachteile der bisherigen arteriellen Thermo- oder Oximetrie/Thermo-Dye-Dilutionskatheter sind aufzulisten, daß die Sensoren (Fiberoptikauge und Thermistor) bisher am distalen Ende angebracht sind und plan am Katheterende neben der Öffnung des Drucklumens enden. Dies hat zur Folge, daß für das Einführen des Meßkatheters immer ein zusätzlicher Einführkatheter verwendet werden muß, durch den der Meßkatheter in das Blutgefäß vorgeschoben wird. Folglich resultiert dieses System aufgrund des größeren Durchmessers in einer deutlich größeren Punktionsfläche und damit Schädigung der Blutgefäßwand und in einem reduzierten Blutfluß.

Des weiteren ist das an der Spitze des Katheters ungeschützt freiliegende Fiberoptikauge des öfteren blind, da die Katheterspitze an gewissen Strukuren der Gefäßwand wie Verzweigungen, atherosklerotischen Veränderungen etc. anliegen bzw. eine Mikrothrombenbildung die Funktion beeinträchtigen kann.

Aus der DE 42 00 030 C2 ist ein Kathetersystem der im Oberbegriff des Patentanspruchs 1 angegebenen Art bekannt. Dieser bekannte Katheter ist vorzugsweise mit einem Dilatationsballon versehen, mittels dessen verengte Gefäße, beispielsweise Herzkranzgefäße, erweitert werden können. Ein Katheter dieser Art wird üblicherweise über eine an der Arteria femoralis des Patienten angebrachte Schleuse eingeführt und über die Aorta des Patienten bis in das Koronarsystem vorgeschoben (vgl. Spalte 4, Zeilen 8 und 9 der DE 42 00 030 C2). Dabei wird der Katheter durch den Führungsdraht im Bereich der Katheterspitze geführt, wobei in dem an die Katheterspitze anschließenden proximalen Bereich des Katheters der Führungsdraht außerhalb des Katheters und parallel zu diesem innerhalb des Blutgefäßes liegt.

Der Führungsdraht dient somit bei dem aus der DE 42 00 030 C2 bekannten Kathetersystem zur Führung der Katheterspitze innerhalb des Blutgefäßsystems. Das Einführen eines solchen Katheters erfordert wegen der Notwendigkeit, einen Einführkatheter oder eine Schleuse zu verwenden, eine vergleichsweise große Punktionsfläche. Wegen der großen Länge des Führungsdrahtes, der üblicherweise mindestens die Länge des Katheters, meist jedoch die doppelte Länge des Katheters aufweist, ist ein großes steriles Operationsfeld erforderlich, da der Führungsdraht während des Einführens leicht mit Gegenständen in der Umgebung des Patienten in Berührung kommen kann.

Aus der EP 0 266 928 A1 ist ein Mehrfunktionskardiovaskulärkathetersystem bekannt, bei dem jedoch die Verwendung eines Führungsdrahtes nicht vorgesehen ist.

### KERN DER ERFINDUNG

Ausgehend von den vorstehend dargelegten Nachteilen und Unzulänglichkeiten der gemäß dem Stand der Technik bekannten Kathetersystemen liegen der Erfindung folgende Aufgaben zugrunde:

Ein neues Kathetersystem soll schnell und einfach in kleinen Blutgefäßen, beispielsweise in der Arteria radialis, mittels direkter, gering traumatisierender Einführungsdrahttechnik plaziert werden können.

Der Katheter soll eine Länge aufweisen, die sicherstellt, daß der Sensor oder die Sensoren auf dem bzw. im Katheter in einem Gefäßgebiet zum Liegen kommen, in dem ein Blutfluß am Katheter vorbei sicher gewährleistet ist und in dem - bei Plazierung im arteriellen System - die Form der Blutdruckkurve der in der Aorta sehr nahe kommt.

Um eine sterile Abdeckung bei der Punktion des Blutgefäßes so klein wie möglich zu machen, soll der verwendete Einführungsdraht so kurz wie möglich sein.

Der Katheter soll unter sterilen Bedingungen positionierbar und repositionierbar bleiben.

Diese Aufgaben werden erfindungsgemäß durch ein Kathetersystem der im Patentanspruch 1 genannten Art gelöst.

Mit dem Kathetersystem gemäß der vorliegenden Erfindung ist die Möglichkeit geschaffen, beispielsweise über eine minimal traumatisierende Punktion der Arteria radialis den Katheter bis in die Nähe der Aorta vorzuschieben, um dort Messungen, beispielsweise Blutdruckmessungen oder optische Spektralmessungen, vorzunehmen, die gegenüber den bisherigen Messungen über die Punktion der Arteria radialis weit überlegen sind.

Das neue Kathetersystem wird wie folgt verwendet: Mittels einer Kanüle wird in üblicher Weise die Arteria radialis punktiert. Sodann wird der kurze Einführungsdraht über das Lumen der Kanüle in das Blutgefäß geschoben und die Kanüle unter Festhalten des Einführungsdrahtes zurückgezogen. Über den Einführungsdraht wird mittels eines Dilatators die Punktionsstelle des Blutgefäßes aufgeweitet. Der Dilatator wird unter Belassung des Einführungsdrahtes im Gefäß ebenso zurückgezogen. Dann wird der Einführungsdraht in die distale Öffnung in der Katheterspitze eingefädelt und der Katheter wird so weit vorgeschoben, daß der Einführungsdraht aus der im Katheterspitzenbereich vorgesehenen seitlichen Öffnung so weit herausragt, daß er sicher mit den Fingern gegriffen werden kann.

Dann wird der Katheter mithilfe des Einführungsdrahtes etwa 5 cm durch Haut- und Unterhautgewebe in das Blutgefäß vorgeschoben. Bevor die seitliche Öffnung des Einführungsdrahtlumens in das Blutgefäß eintritt, wird der Einführungsdraht gezogen. Der Katheter wird danach (ohne Einführungsdraht) weiter bis etwa 50 cm vorgeschoben, so daß er mit der Spitze eine große Arterie (beispielsweise die Arteria axillaris) erreicht. Das Anbringen des Katheters ist minimal traumatisierend, da nur die leicht zugängliche Arteria radialis punktiert werden muß und die Verwendung einer Schleuse nicht erforderlich ist. Dank der Kürze des Einführungsdrahtes kann das sterile Feld sehr klein gehalten werden, ohne daß die Gefahr einer Kontamination des Einführungsdrahtes während des Einführens besteht.

Im Gegensatz zum Stand der Technik gemäß der DE 42 00 030 C2 dient der erfindungsgemäß vorgesehene kurze Einführungsdraht nicht zur Führung des Katheters innerhalb des Blutgefäßsystems, sondern vielmehr nur zum Einführen des Katheters durch die Punktionsstelle in das Blutgefäßsystem. Der erfindungsgemäß vorgesehene kurze Einführungsdraht ersetzt daher die zum Einführen von Kathetern gemäß der DE 42 00 030 C2 üblicherweise erforderliche Schleuse oder Einführungskanüle. Er hat somit mit dem gemäß der DE 42 00 030 C2 vorgesehenen Führungsdraht nur formale Ähnlichkeit, erfüllt jedoch einen vollkommen anderen Zweck: Der Führungsdraht gemäß der DE 42 00 030 C2 weist eine Länge auf, die mindestens gleich der Länge des Katheters ist, da er dazu dient, einen Katheter längs des Führungsdrahtes auch in kleine Blutgefäße beispielsweise in das Konorarsystem einzuführen (vgl. Spalte 4, Absatz 1 der DE 42 00 030 C2).

Die abhängigen Patentansprüche 2 bis 10 betreffen vorteilhafte Ausführungsformen des Kathetersystems gemäß Patentanspruch 1.

Weitere Ausgestaltungen, Merkmale und Vorteile der vorliegenden Erfindung werden nachstehend anhand des in den Figuren 1 bis 4 exemplarisch veranschaulichten Ausführungsbeispiels näher erläutert.

Es zeigt:

### KURZE BESCHREIBUNG DER ZEICHNUNGEN

- Figur 1:: eine (stark vergrößert dargestellte) Längsschnittansicht eines Ausführungsbeispiels eines Kathetersystems gemäß der vorliegenden Erfindung;
- Figur 2:: eine Querschnittansicht des Kathetersystems aus Figur 1 entlang der Schnittlinie II - II;
- Figur 3:: eine Kanalteilung am proximalen Ende des intravasalen Teils des Kathetersystems aus Figur 1 mit daran angeflanschten Gerätschaften; und
- Figur 4:: die Kanalteilung des Kathetersystems aus Figur 1 mit Klebefolie und mit Schutzschlauch.

### DETAILLIERTE BESCHREIBUNG DER ZEICHNUNGEN

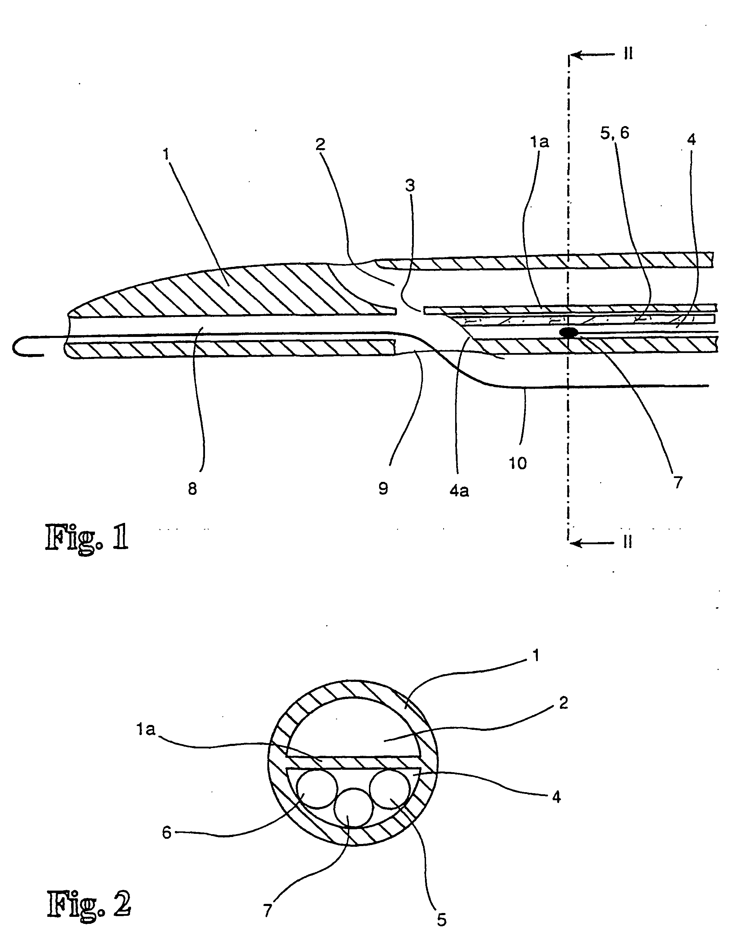

Gleiche oder ähnliche Teile sind in den Figuren 1 bis 4 mit den gleichen Bezugszeichen versehen.

Das anhand der Figuren 1 bis 4 exemplarisch veranschaulichte Kathetersystem gemäß der vorliegenden Erfindung weist bei normalgewichtigen und normalgroßen erwachsenen Personen eine nutzbare intravasale Länge von etwa 50 cm um einen Durchmesser von etwa 1,3 mm auf. Der in diesem Ausführungsbeispiel zweilumige Katheterschaft 1, 1a ist idealerweise aus gering thrombogenem Material wie beispielsweise Polyurethan hergestellt.

In einem Lumen 4 (= sogenanntes Sensorlumen 4) des Katheterschaftes 1, 1a werden die Sensorleitungen, beispielsweise Fiberoptik- und/oder Thermistor-Anschlußleitungen, geführt; das andere Lumen 2 (= sogenanntes Druck- und Blutentnahmelumen 2) dient zur Blutentnahme und Druckmessung über eine Flüssigkeitssäule. Des weiteren kann der Katheterschaft 1, 1a Längenmarkierungen und einen Röntgenkontraststreifen aufweisen.

Der Katheter weist distal ein konisch angeformtes Einführungsdrahtlumen 8 auf, durch das ein Einführungsdraht 10 geschoben werden kann, der deutlich kürzer als der gesamte Katheter ist, das heißt in diesem Beispiel etwa 20 cm. Das Einführungsdrahtlumen 8 führt etwa 5 cm nach der Spitze durch eine seitlich vorgesehene Öffnung 9 wieder ins Freie. Durch diese Öffnung 9 gelangt der Einführungsdraht 10 nach distalem Einführen wieder ins Freie.

An diese - ausreichend große - Öffnung 9 proximal angrenzend werden die Sensoren, beispielsweise Fiberoptikauge 4a und Thermistor 5, 6, angebracht. Die plane Fläche des Fiberoptikauges 4a ist zum Katheterschaft 1, 1a unter einem Winkel von kleiner als neunzig Grad angeordnet, wodurch die Lichtemission und die Lichtreflexion in bzw. aus dem am Katheter vorbeiströmenden Blut gewährleistet ist. Mittels eines Durchbruchs 3 zum Drucklumen im Katheterschaft 1, 1a gelangt kontinuierlich heparinisierte Spüllösung an das Fiberoptikauge 4a, um Gerinnselbildung an letzterem zu vermeiden.

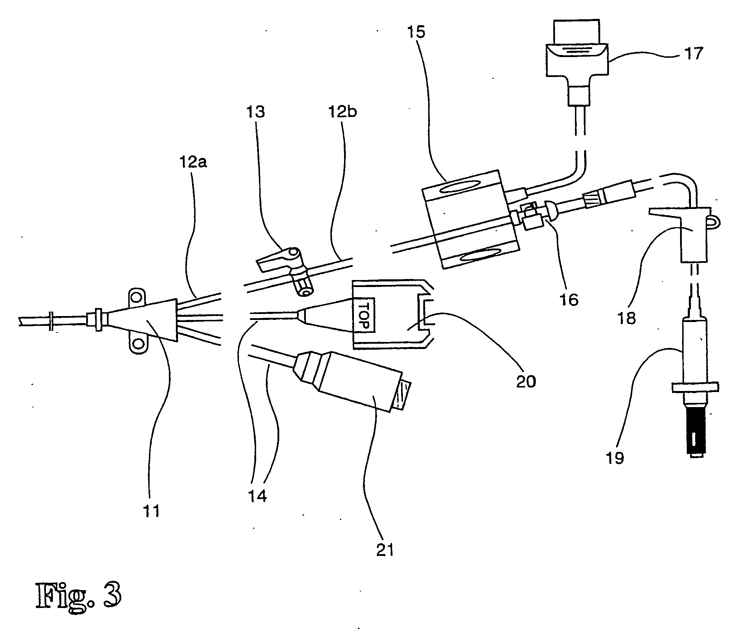

Auf der kontralateralen Seite des Katheters findet sich etwa 3 cm hinter der Spitze die Öffnung des mittels heparinisierter Spüllösung kontinuierlich gespülten Druck- und Blutentnahmelumens 2. Wie aus der Darstellung der Figur 3 ersichtlich ist, befinden sich am proximalen Ende des intravasalen Teils des Katheters eine Kanalteilung 11 und - daran angeflanscht - beispielsweise etwa 10 cm lange Kanalverlängerungen für den Abschnitt 12a eines Druckschlauchs 12a, 12b und für Sensoren 14, in diesem Fall ein Anschluß mit Fiberoptikstecker 20 und ein weiterer Anschluß mit Thermistorstecker 21. Der Abschnitt 12a des Druckschlauchs 12a, 12b mündet in einen in die Schlauchleitung eingeflanschten Dreiwegehahn 13 mit einem seitlichen Luer-Lock-Anschluß.

An diesem Dreiwegehahn 13 kann ein zusätzlicher Druckschlauch zum leichteren Nullpunktabgleich eines Druckaufnehmersystems angeschlossen sein. Proximal an diesen Dreiwegehahn 13 ist ein weiterer, etwa 10 cm langer Abschnitt 12b des Druckschlauchs 12a, 12b angeflanscht, der direkt an einen elektronischen Druckaufnehmer 15 mit geringer Compliance angeflanscht ist. Durch die festen Flanschverbindungen werden Luftansammlungen an den sonst üblichen Konnektoren deutlich reduziert. Die gesamte Innenseite des Druck- und Blutentnahmelumens 2 vom Katheter bis zum Druckaufnehmer 15 weist eine möglichst durchgehend glatte innere Oberfläche auf.

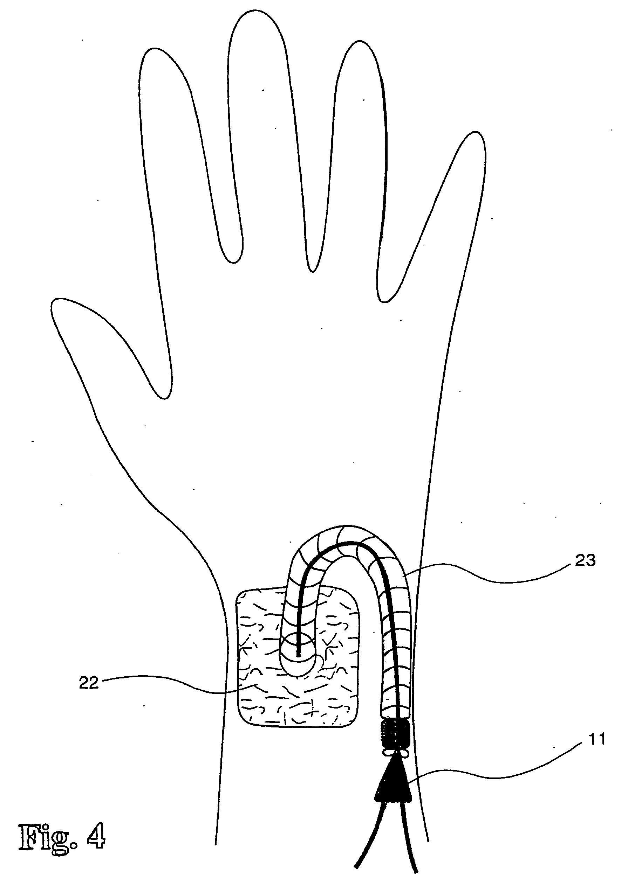

Wie aus der Darstellung der Figur 4 ersichtlich ist, ist direkt an der Kanalteilung 11 des Katheters eine sterile, transparente und hochadhäsive Klebefolie 22 (sogenannte Schutzschlauchklebefolie 22) mit einem etwa 10 cm langen integrierten Schutzschlauch 23 angebracht. Hiermit wird eine optimale Erstpositionierung sowie Repositionierung des Katheters bei Sensorfehllage ermöglicht.

Es sei an dieser Stelle besonders darauf hingewiesen, daß der kurze Einführungsdraht 10 von etwa 20 cm Länge ein wesentlicher Bestandteil des Kathetersystems ist. Dieser Einführungsdraht 10 weist einen auf das kurze Einführungsdrahtlumen 8 des Katheters adaptierten Durchmesser auf. Dazu beigepackt sind eine Kanüle mit einem auf den Einführungsdraht 10 adaptierten Innendurchmesser sowie eine Spritze und ein sogenannter Dilatator von etwa 5 cm Länge und von etwa 1,3 mm Durchmesser.

Die Anwendung des in den Figuren 1 bis 4 exemplarisch veranschaulichten Kathetersystems gemäß der vorliegenden Erfindung wird nachfolgend erläutert:

Zunächst wird das Blutentnahme-/Druckmeßsystem des Katheters sowie der Druckschlauch 12a, 12b für den Nullabgleich (optional angeschlossen an den Dreiwegehahn 13) mit Flüssigkeit (beispielsweise heparinisierte physiologische Kochsalzlösung) über ein Spülventil 16, über einen Durchflußregler 18 und über eine Tropfkammer 19 gefüllt und der elektrische Druckaufnehmer 15 mittels eines Verbindungssteckers 17 an einen Druckmonitor angeschlossen (vgl. Figur 3).

Mittels einer Kanüle wird in üblicher Weise die Arteria radialis punktiert. Sodann wird der kurze Einführungsdraht 10 über das Lumen der Kanüle in das Blutgefäß geschoben und die Kanüle unter Festhalten des Einführungsdrahtes 10 im Gefäß zurückgezogen. Über den Einführungsdraht 10 wird mittels des Dilatators die Punktionsseite des Blutgefäßes aufgeweitet. Der Dilatator wird unter Belassung des Einführungsdrahtes 10 im Gefäß ebenso zurückgezogen. Sodann wird der Einführungsdraht 10 in die distale Öffnung in der Katheterspitze eingefädelt und über das kurze Einführungsdrahtlumen 8 soweit vorgeschoben, daß der Einführungsdraht 10 wieder weit genug aus dem etwa 5 cm von der Spitze zurückliegenden Lumen ausgetreten ist, damit er sicher mit den Fingern gegriffen werden kann.

Dann wird der Katheter mithilfe des Einführungsdrahtes 10 etwa 5 cm durch Haut und Unterhautgewebe in das Blutgefäß vorgeschoben. Der Einführungsdraht 10 wird gezogen. Danach wird der Katheter weiter bis zu 50 cm vorgeschoben, bis er mit der Spitze in einer großen Arterie (beispielsweise der Arteria axillaris) zum Liegen kommt, was auch an der Form der simultan über das Druck- und Blutentnahmelumen 2 registrierten Blutdruckkurve erkannt werden kann.

Zum Nullpunktabgleich wird der Dreiwegehahn 13 zur Atmosphäre hin geöffnet und mittels Heben des Armes auf Herzhöhe gebracht. Wenn ein Nullpunktabgleich durch Heben des Armes nicht möglich ist, wird durch Anschließen eines mindestens 50 cm langen Druckschlauchs an dem Luer-Lock-Anschluß des Dreiwegehahns 13 eine der Herzhöhe entsprechende, zur Atmosphäre hin offene Flüssigkeitssäule erzeugt und der Nullpunktabgleich vorgenommen.

Das Kathetersystem gewährleistet die sichere Erfassung von physiologischen bzw. biochemischen Parametern über Plazierung der Sensoren im aortennahen Strömungsgebiet beim Einführen des Katheters über eine kleine Arterie, vorzugsweise die Arteria radialis. Das beschriebene Ausführungsbeispiel des Kathetersystems gemäß der vorliegenden Erfindung ermöglicht die aortennahe Druckmessung (Rulf et al, 1990; van Beck et al, 1993) und damit auch die sichere Auswertung des Drucksignals nach der Pulskonturmethode, die sichere Erfassung von transkardiopulmonalen Indikatorverdünnungskurven und die kontinuierliche Sauerstoffsättigungsmessung. Das Prinzip ist auf die Messung anderer Parameter jederzeit ausdehnbar.

Der Katheter kann in oberflächennah liegende, periphere Arterien mittels eines kurzen Einführungsdrahtes 10 und über ein kurzes Einführungsdrahtlumen 8 eingeführt werden. Durch diese sichere und einfache Methode entfällt die bisher notwendige Benutzung eines Kathetereinführsystems. Bei gleichem Meßkatheterdurchmesser wird damit ermöglicht, daß der Katheter in kleinere Arterien, beispielsweise die Arteria radialis, eingeführt werden kann. Durch diese neue Technik mit kurzem Führungsdraht 10 kann das sterile Feld, das für das Einführen geschaffen werden muß, wesentlich kleiner gestaltet werden, als das bei Verwendung herkömmlicher Einführsets mit langen Führungsdrähten notwendig wäre.

Die von der Katheterspitze zurückversetzte Anordnung der Sensoren gewährleistet, daß diese immer optimal im strömenden Blut plaziert werden können und nicht durch gewisse Gefäßstrukturen in ihrer Funktion gestört werden. Des weiteren kann über den Durchbruch 3 im Katheterschaft 1, 1a eine direkte Spülung des Fiberoptikauges 4a durchgeführt werden.

Durch die direkte Anflanschung eines Druckaufnehmers 15 an den Meßkatheter wird eine optimale Druckmessung über ein relativ dünnes Drucklumen ermöglicht, da durch die weitestgehend glatte innere Oberfläche des Drucklumens bis zum elektrischen Druckaufnehmer 15 die Ansammlung von das Drucksignal dämpfenden Luftblasen vermieden wird.

Die Erfahrung mit manchen Sensorsystemen (beispielsweise Fiberoptik) zeigt, daß für ideale Meßvorgänge von Zeit zu Zeit eine Korrektur der Katheterlage erforderlich sein kann. Sowohl dies als auch die optimale Erstpositionierung des Kathetersystems werden durch die integrierte Schutzschlauchklebefolie 22 gewährleistet.

## Patentansprüche

1. Kathetersystem mit einem Einführungsdraht (10) und einem Katheter, bei dem im distalen Katheterspitzenbereich ein Einführungsdrahtlumen (8) vorgesehen ist, das sich von der Katheterspitze bis zu einer seitlich im Katheterspitzenbereich vorgesehenen Öffnung (9) erstreckt, **dadurch gekennzeichnet, daß** der Einführungsdraht (10) eine Länge aufweist, die deutlich kleiner als die Länge des Katheters ist, wobei die Länge des Einführungsdrahtes (10) so bemessen ist, daß dieser nur zum Einführen des Katheters durch eine Punktionsstelle in ein Blutgefäß, nicht jedoch zum Führen des Katheters innerhalb des Blutgefäßes dient.

2. Kathetersystem nach Anspruch 1, **dadurch gekennzeichnet, daß** mindestens zwei Lumina (2, 4) vorgesehen sind, von denen ein erstes Lumen (2) einen im Katheterspitzenbereich liegenden seitlichen Ausgang aufweist und daß ein zweites (4) der mindestens zwei Lumina (2, 4) einen optisch durchlässigen Verschluß (4a) aufweist, der proximal an die Öffnung (9) angrenzt.

3. Kathetersystem nach Anspruch 2, **dadurch gekennzeichnet, daß** der Ausgang des ersten Lumen (2) und die Öffnung (9) bei etwa der gleichen Axialposition angeordnet sind.

4. Kathetersystem nach Anspruch 3, **dadurch gekennzeichnet, daß** ein Durchbruch (3) vorgesehen ist, der eine Verbindung des ersten Lumens (2) zu der Öffnung (9) herstellt.

5. Kathetersystem nach einem der Ansprüche 2 bis 4, **dadurch gekennzeichnet, daß** vorgesehen sind:
a) ein Druckschlauch (12a/12b), der mit der proximalen Endöffnung des ersten Lumen (2) verbunden ist;
b) Stränge einer Fiberoptik (5, 6), die im zweiten Lumen (4) angeordnet sind, unmittelbar an dem Verschluß (4a) enden und an der proximalen Endöffnung des Lumen (4) austreten;
c) und ein thermisches Sensorelement (7), das in dem zweiten Lumen (4) im Bereich des Verschlusses (4a) angeordnet ist und dessen Leitungsverbindung aus der proximalen Endöffnung des zweiten Lumen (4) austritt.

6. Kathetersystem nach Anspruch 5, **dadurch gekennzeichnet, daß** der Druckschlauch zwei Druckschlauchabschnitte aufweist und daß zwischen den Druckschlauchabschnitten (12a und 12b) ein Dreiwegehahn mit seitlichem Luer-Lock-Anschluß angeordnet ist.

7. Kathetersystem nach Anspruch 5, **dadurch gekennzeichnet, daß** einer der Druckschlauchabschnitte (12b) mit einem elektrischen Druckaufnehmer und über einen Verbindungsstecker (17) mit einem Monitor verbunden ist.

8. Kathetersystem nach einem der Ansprüche 6 bis 7, **dadurch gekennzeichnet, daß** einer der Druckschlauchabschnitte (12b) über ein Spülventil (16) mit einem Durchflußregler (18) und gegebenenfalls einer Tropfkammer (19) verbunden ist.

## Claims

1. Catheter system comprising an insertion wire (10) and a catheter, in which there is provided in the distal catheter tip region an insertion wire lumen (8), which extends from the catheter tip up to an opening (9) provided laterally in the catheter tip region, **characterized in that** the insertion wire (10) has a length that is markedly smaller than the length of the catheter, wherein the length of the insertion wire (10) is so dimensioned that said insertion wire is used only to insert the catheter through a puncture site into a blood vessel but not to guide the catheter inside the blood vessel.

2. Catheter system according to claim 1, **characterized in that** at least two lumina (2, 4) are provided, of which a first lumen (2) has a lateral outlet situated in the catheter tip region, and that a second (4) of the at least two lumina (2, 4) has an optically transparent closure (4a), which proximally adjoins the opening (9).

3. Catheter system according to claim 2, **characterized in that** the outlet of the first lumen (2) and the opening (9) are disposed at approximately the same axial position.

4. Catheter system according to claim 3, **characterized in that** a through-hole (3) is provided, which establishes a connection of the first lumen (2) to the opening (9).

5. Catheter system according to one of claims 2 to 4, **characterized in that** the following are provided:
a) a pressure tube (12a/12b), which is connected to the proximal end opening of the first lumen (2);
b) fibre optic strands (6, 7), which are disposed in the second lumen (2), terminate directly at the closure (4a) and exit at the proximal end opening of the lumen (4);
c) and a thermal sensor element (7), which is disposed in the second lumen (4) in the region of the closure (4a) and the line connection of which exits from the proximal end opening of the second lumen (4).

6. Catheter system according to claim 5, **characterized in that** the pressure tube comprises two pressure tube portions and that disposed between the pressure tube portions (12a and 12b) is a three-way tap with a lateral Luer lock connector.

7. Catheter system according to claim 5, **characterized in that** one of the pressure tube portions (12b) is connected to an electrical pressure pick-up and by a plug-in connector (17) to a monitor.

8. Catheter system according to one of claims 6 to 7, **characterized in that** one of the pressure tube portions (12b) is connected by an irrigation valve (16) to a flow regulator (18) and optionally to a drip chamber (19).

## Revendications

1. Système de cathéter pourvu d'un fil d'introduction (10) et d'un cathéter, où une lumière pour le fil d'introduction (8) est prévue dans la zone distale pointue du cathéter qui s'étend de la pointe du cathéter jusqu'à une ouverture (9) ménagée latéralement au niveau de la pointe du cathéter, **caractérisé en ce que** le fil d'introduction (10) a une longueur nettement inférieure à celle du cathéter, la longueur du fil d'introduction (10) étant telle qu'il ne sert qu'à l'introduction d'un cathéter dans un vaisseau sanguin par un point de ponction et non au guidage du cathéter dans le vaisseau sanguin.

2. Système de cathéter selon la revendication 1, **caractérisé en ce qu'**au moins deux lumières (2, 4) sont prévues, dont une première lumière (2) présentant une sortie placée latéralement dans la zone de la pointe du cathéter et une deuxième (4) des au moins deux lumières (2, 4) présentant un bouchon translucide (4a) touchant l'ouverture en position proximale (9).

3. Système de cathéter selon la revendication 2, **caractérisé en ce que** la sortie de la première lumière (2) et l'ouverture (9) ont approximativement la même position axiale.

4. Système de cathéter selon la revendication 3, **caractérisé en ce qu'**un passage est ménagé (3) qui établit une liaison entre la première lumière (2) et l'ouverture (9).

5. Système de cathéter selon l'une des revendications 2 à 4, **caractérisé en ce que** sont prévus :
a) un tuyau résistant à la pression (12a/12b) connecté à l'orifice terminal proximal de la première lumière (2) ;
b) des brins d'une fibre optique (5, 6) placés dans la deuxième lumière (4) se terminant directement au niveau du bouchon (4a) et ressortant à l'extrémité terminale proximale de la lumière (4) ;
c) et un capteur thermique (7), placé dans la deuxième lumière (4) à proximité du bouchon (4a) et dont le fil de liaison ressort de l'orifice terminal proximal de la deuxième lumière (4).

6. Système de cathéter selon la revendication 5, **caractérisé en ce que** le tuyau résistant à la pression est formé de deux tronçons et qu'entre ces tronçons de tuyau résistant à la pression (12a et 12b) est placé un robinet trois voies muni d'un raccord Luer-lock latéral.

7. Système de cathéter selon la revendication 5, **caractérisé en ce qu'**un des tronçons du tuyau résistant à la pression (12b) est connecté à un capteur électrique de poussée et à un moniteur par le biais d'une fiche de connexion (17).

8. Système de cathéter selon l'une des revendications 6 ou 7, **caractérisé en ce qu'**un des tronçons résistant à la pression (12b) est relié par une soupape de vidange (16) à un régulateur d'écoulement (18) et, le cas échéant, à une chambre compte-gouttes (19).
